# EUROPEAN PATENT APPLICATION

(11) **EP 2 174 691 A1**
(43) Date of publication of application: **14.04.2010**
(21) Application number: 08166380.9
(22) Date of filing: 10.10.2008
(51) Int. Cl.: A61Q 19/00, A61Q 1/00, A61Q 1/02, A61K 8/02

(54) **Cosmetic core stick with a water based hard core part**

(71) Applicant: Coty Deutschland GmbH, 55116 Mainz (DE)
(72) Inventor: The designation of the inventor has not yet been filed
(74) Representative: Ziebig, Marlene

(57) **Abstract**

The present invention relates to a cosmetic core stick consisting of an anhydrous shell part comprising at least one wax component, polyethylene, at least one skin conditioning agent, at least one colour additive, at least one spherical powder, at least one UV filter substance, at least one organic filler and at least one surfactant and a water based hard core part which is a solid W/O or W/Si emulsion comprising water, polyethylene, at least one emollient, at least one cosmetically or pharmaceutically water and/or oil soluble active, at least one W/O or W/Si emulsifier, at least one skin moisturizing agent, at least one colour additive and at least one preservative.

## Description

The present invention relates to a cosmetic core stick consisting of an anhydrous shell part and a water based hard core part. The anhydrous shell part is a fatty stick composition used for covering and concealing skin anomalies. The water based hard core part comprises high contents of water and water soluble cosmetically and/or pharmaceutically active ingredients to nourish and care for the covered skin. In particular, the present invention relates to concealing sticks, foundation sticks, moisturizing sticks, anti-acne sticks, anti-blemish sticks, sticks for the prophylaxis and treatment of wrinkles and sunscreen sticks, skin firming and lifting sticks, sticks for nourishing and soothing skin.

Cosmetic sticks are known in the state of the art. Usually stick formulations are anhydrous fatty mixtures of solid or semi-solid waxes and liquid oils, where the highly purified paraffin oils and waxes represent the stick base mass and provides the stability of the stick at room temperature. Although these sticks are often widely used as lipsticks or concealing sticks they often result in a greasy unpleasant feeling on the skin and dermatological diseases such as acne are made worse if pores are clogged. Further, oily and waxy materials tend to soil clothing and other items with which they are brought into contact with both the fatty material itself and the colour particles usually solved therein.

To overcome this problem GB 2 014 852 provides a lipstick having a core sheath construction comprising at least one viscous oil and one wax in each part of the stick. By varying the viscosity of the oily component in the two phases the spreading properties, the adhesion to the lips and the sensation on the skin was determined. Preferably a low-viscosity oily ingredient was used in the external part of the stick and a high-viscosity oily ingredient was used in the internal part of the stick composition.

US 6,358,498 discloses a two part stick composition wherein the inner part contains the cosmetic active ingredients and the outer part forms a wax free shell which does not transfer pigments through brief contact. The outer shell comprises at least a solid protein, a liquid polyol, a solid polyol and one emollient.

US 4,334,546 disclose a cosmetic pencil for a wax-like cosmetic material consisting of polyethylene and fully hydrogenated or partly hydrogenated castor oil. The toughness of the resulting pencil is sufficient enough to be sharpened with a conventional pencil sharper and the properties are not affected by the inner cosmetic compositions.

It is a disadvantage of the sticks mentioned above that only oil soluble compounds can be formulated in the stable cosmetic compositions. US 2004/0258721 discloses a water-in-oil emulsion which can be prepared as a stable stick, if solid and high-melting waxes are used. However, many active ingredients are temperature sensitive and cannot be used in high melting compositions.

It would be desirable to have a cosmetic composition which solves both hydrophilic and lipophilic cosmetically and/or pharmaceutically ingredients and which can be produced at low temperatures so that temperature sensitive substances can be included. The composition should be stable at room temperature and after administration to the skin the composition should satisfy the aesthetic requirements of the consumers. The feeling on the skin should be natural and comfortable.

According to the invention a cosmetic core stick is provided which consists of an oily and an aqueous phase to solve hydrophilic and lipophilic active ingredients needed for concealing and caring for biological surfaces, in particular human skin.

Particularly, the present invention provides a cosmetic core stick consisting of
a) an anhydrous shell part comprising at least one wax component, polyethylene, at least one skin conditioning agent, at least one colour additive, at least one spherical powder, at least one UV filter substance, at least one organic filler and at least one surfactant
   and
b) a water based hard core part which is a solid W/O or W/Si emulsion comprising water, polyethylene, at least one emollient, at least one cosmetically or pharmaceutically water and/or oil soluble active, at least one W/O or W/Si emulsifier, at least one skin moisturizing agent, at least one colour additive and a preservative.

That means the cosmetic core stick of the invention combines the properties of a fatty stick provided as the shell coat part with the properties of a water-in-oil or water-in-silicone emulsion provided as the water based hard core part. Combining the advantages of both parts a cosmetic product is provided which meets the requirements of several cosmetic substances usually applied separately.

For example pearlescent pigments which are highly shear-sensitive, which means that the pearlescent effect fails to appear after shearing cannot be used in water-containing formulations usually. Further, unstable formulations would arise. According to the invention this problem is solved by mixing the pearlescent pigments in the anhydrous shell part.

According to the invention it is also possible to incorporate water-soluble ingredients into the cosmetic stick formulation. In particular most of anti-acne, anti-wrinkles, anti-aging ingredients which are needed to improve the skin health are only water soluble and many of them are temperature sensitive. For instance antioxidants with the ability to scavenge free radicals helps to protect the skin from environmental damage and even out the skin tone are often temperature sensitive. Further, the majority of plant, nut, seed, marine extracts possessing wide spectrum of important properties such as protective, detoxifying, revitalizing tired skin, adjusting moisture balance, anti-bacterial, anti-irritant, anti-microbial, anti-inflamatory, anti-pruritic, skin lifting are very often heat sensitive as well.

This is the main advantage of the internal water-based part. Further advantages are related to the product aesthetics: the stick provides better gliding, end uniformity, better actives delivery, pleasant feel.

The anhydrous shell part provides the properties of a classical concealer or a foundation. The composition is a cream to powder formula. The shell part is based on polyethylene and a wax component. The mixture is highly stable at room temperature and forms a suitable framework for the functional ingredients. The melting point of the shell part is determined by the melting points of the wax components and the molecular weight and the number of branches of the polyethylene. Preferably the shell part has a melting point of at least 60° C, more preferred between 65 °C and 70 °C and most preferred between 75 °C and 80 °C.

Concealer or foundations are applied to the skin to hide pores, imperfections, fine lines and the like. Thus, colour additives having a high opacity are contained in the shell part according to the invention. Spherical powders are further added to improve the product application by providing oil absorption, a good spreading ability, a creamy feeling and a blurring and soft focus effect. In addition, fine lines and wrinkles are filled with the spherical powders.

A concealer or a foundation stick according to the invention is also applied to moisturize the skin, to balance the oil level of the skin, and to provide protection against the adverse effects of sunlight, wind, and other environmental factors. Thus the shell part of the cosmetic core stick according to the invention contains at least a skin conditioning agent and a UV-filter component.

The inner core of the cosmetic stick of the invention is a solid W/O or W/Si emulsion with a high content of water. It was found that stable W/O or W/Si emulsion can be achieved without using any wax components. The polyethylene alone stabilizes the structure of the water based hard core part. Thereby the molecular weight and the branching of the polyethylene determines the melting temperature and the hardness of the inner core part. Preferably the melting temperature of the water based core part is in the range of 45 °C to 65 °C, more preferred in the range of 50 °C to 60 °C and most preferred in the range of 55 °C to 58 °C. Surprisingly it was achieved to formulate a stable water based hard core part with these low melting temperatures. Due to the low melting temperature even cosmetically or pharmaceutically water soluble actives which are temperature sensitive can be included into the inner core part. For instance antioxidants, such as phenolic antioxidants, butylated or hydroxyanisol, butylated hydroxytoluene, propyl gallate, mono-tertiary-butylhydroquinone, vitamins, such as vitamin C, vitamin F, vitamin A, vitamin B, vitamin E or vitamin H, bioactive complexes, such as vegetable proteins, adenosine triphosphate, sodium chondroitin sulfate, glycine, alanine, proline, creatine, riboflavin, niacinamide, acetyl tyrosine etc., extracts, for instance beta vulgaris root extract, haberlea rhodopensis leaf extract, faex yeast extract etc., some natural preservatives, preferably biopein, neopein, suprapein etc. or heat-sensitive dyes can be used in the inner core part of the present invention. It is further possible to include oil soluble cosmetically or pharmaceutically active substances into the water based hard core part according to the invention. This is very surprising as the inner core part does not contain any waxes.

The water based hard core part contains a high content of water and additionally a high content of skin moisturizing agents to moisturize the skin effectively. According to the invention it is possible to achieve stable compositions comprising up to 85 percent by weight of water. In a preferred embodiment the water based hard core part contains more than 50 percent by weight, preferably more than 60 percent by weight, more preferred more than 70 percent by weight and most preferred more than 80 percent by weight of water based on total weight of the water based hard core part. This stability is achieved by combining the polyethylene, the emollient and the emulsifier according to the invention. The resulting stick is hard but not brittle. The composition is highly spreadable and pleasant on the skin after application.

The cosmetic core sticks according to the invention are solid at room temperature and are not sensitive to shear forces. They further remain stable and spreadable within the entire temperature range from 5°C to 50°C. Long time stability is also provided by the cosmetic core sticks according to the invention. The sticks remain stable for more than 12 month at 45 °C.

In a preferred embodiment the cosmetic core stick according to the invention comprises cosmetically acceptable auxiliary substances in both parts, the anhydrous shell part and the water based hard core part. As a cosmetically acceptable auxiliary substance any additive can be used which improves the quality of the cosmetic composition. This include for example powders, such as absorbers or thickening agents, consistency-imparting agents, such as viscosity control agents, fillers, film forming agents, solvents, preservatives, fragrances, such as perfume oils, sun filters, photoreactive agents, stabilizers, repellents, wound healing agents and mixtures thereof.

The waxes that may be used in the anhydrous shell part cosmetic composition of the invention are cosmetically acceptable compounds that are solid at room temperature. They are intended to structure the composition in particular in stick form. Suitable compounds may be hydrocarbon-based waxes and/or silicone waxes, in particular of plant, mineral, animal and/or synthetic origin. In a preferred embodiment the waxes are solid and high melting waxes having a melting point of greater than 60 °C, more preferred greater than 70 °C and most preferred still greater than 80 °C.

All waxes which are generally used in personal care/cosmetic industry can be used. Suitable natural waxes are for instance beeswax, preferably PEG8-Beeswax, carnauba wax, candelilla wax, ouricoury wax, Japan wax, cork fibre wax, espartograss wax, sugarcane wax, rice wax, blossom wax, leaf waxes of softwoods, coffee wax, flax wax, sesame wax, fruit waxes, montan wax, paraffin, microcrystalline wax or ceresin. Suitable synthetic waxes are for instance ozokerite, mineral waxes or emulsifying waxes or alternatively fatty acid esters, for instance octacosanyl stearate or glycerides provided that they are solid until the required temperature. In a preferred embodiment of the invention ozokerite wax is used in the anhydrous shell part of the cosmetic core stick.

The waxes are contained at about 0.1 to about 20 weight %, preferably at about 0.1 to about 15 weight %, most preferred at about 0.1 to about 10 weight % based on the total weight of the anhydrous shell part of the composition.

The cosmetic core stick of the invention contains polyethylene in both parts of the composition. Linear and/or branched polyethylene can be used as a single compound or as a mixture of several polyethylene compounds. In one embodiment of the invention in the anhydrous shell part of the composition a polyethylene with a molecular weight lower than 526^{g}/ₘₒₗ, in particular from 475^{g}/ₘₒₗ to 525^{g}/ₘₒₗ, preferably lower than 476^{g}/ₘₒₗ, in particular from 425^{g}/ₘₒₗ to 475^{g}/ₘₒₗ, most preferred lower than 426^{g}/ₘₒₗ, in particular from 325^{g}/ₘₒₗ, to 425^{g}/ₘₒₗ is used. The melting point of the polyethylene used for the anhydrous shell part composition is preferably in the range from 65° C to 85° C. In particular Jeenate 3H, 4H and/or 5H polyethylene is used according to the invention.

The anhydrous shell part contains the polyethylene in an amount of 0.1 to 35 percent by weight, preferably 8 to 15 percent by weight based on total weight of the anhydrous shell part.

In one embodiment of the invention the water based hard core part contains a polyethylene with a molecular weight higher than 174^{g}/ₘₒₗ, in particular from 175g/ₘₒₗ to 225^{g}/ₘₒₗ, preferably higher than 249^{g}/ₘₒₗ, in particular from 250^{g}/ₘₒₗ to 300^{g}/ₘₒₗ, most preferred higher than 324^{g}/ₘₒₗ, in particular from 325^{g}/ₘₒₗ to 375^{g}/ₘₒₗ. The melting point of the polyethylene used for the water based hard core part is preferably in the range from 50 °C to 70 °C, more preferred in the range from 60 °C to 68 °C. In particular a linear polyethylene, such as Jeenate 3H polyethylene is preferably used according to the invention.

The water based hard core part contains the polyethylene in an amount of 0.1 to 30 percent by weight, preferably 1 to 20 percent by weight, most preferred 5 to 15 percent by weight based on total weight of the water based hard core part.

In a preferred embodiment both parts of the cosmetic core stick contain the same polyethylene, in particular Jeenate 3H. Very good results can be reached by using a combination of low molecular weight polyethylenes (for example Jeenate 2H and Jeenate 3H). Thus, a hard core stick with a low melting point (as low as 55°C) and excellent slip is formed.

The polyethylene used according to the invention, in particular the soft polyethylene used in the water based hard core part provides a better skin feel, more pay off and an ease in manufacturing, in particular by decreasing the pouring temperature.

A suitable skin conditioning agent which is contained in the anhydrous shell part is every agent that attracts and holds moisture in the skin, in particular vitamins, mineral salts, trace elements, plant extracts, animal extracts, proteins, peptides, amino acids, enzymes, glycosaninoglycans, capric/caprylic triglycerides, esters of fatty acids, for example cetearyl octanoate (Jeechem CEO) or neopentyl glycol diethylhexanoate (Schercemol NGDO) and mixtures thereof. Other light conditioning agents can be used as well: isopropyl isostearate, lauryl lactate, octyl pelargonate, octyl isononanoate, diisopropyl sebacate, neopentyl glycol dioctanoate, cetyl ethylhexanoate, ethylhexyl isononanoate, neopentyl glycol dicaprate, isodecyl oleate, isostearyl neopentanoate, glyceryl trioctanoate, isocetyl stearate, isostearyl isostearate, diasoproyl dimer dilineoleate, PPG-3 benzyl ether myristate, isopropyl palmitate, isopropyl miristate or octyl palmitate.

According to a preferred embodiment of the invention high amounts of skin conditioning agents are contained in the anhydrous shell part of the core stick composition. The amount of skin conditioning agent is in the range from 0.1 to 50 weight %, more preferred from 10 to 40 weight % most preferred from 25 to 35 weight %.

According to the invention the water based hard core part contains at least one skin moisturizing agent. Moisturizers not only increase the skin's water content, but they also protect the skin and encourage an orderly desquamation (shedding) process that makes the skin appear more smooth.

Skin moisturizing agents are occlusives such as silicones and derivatives thereof, emollients and humectant agents. Suitable silicones according to the invention are for instance dimethicone or cyclomethicone. Suitable humactants are glycerol, sorbitol, mannitol, glycine, chitosan, fucogel, propylene glycol, polypropylene glycol, polyethylene glycol, dipropylene glycol, butylene glycol, alpha-hydroxy acids, such as lactic acid, glycolic acid, glutamic acid, glucoronic acid, hyaluronic acid, sodium pyrrolidone carboxylic acid, salts of the given acids, ascorbyl glucoside, pentylene glycole (and) Tamarindus Indica Extract, urea and salts of metals of the first and second main group.

In a preferred embodiment humactants, in particular salts of acids, such as sodium hyaluronate or glycols, for instance polyethylene glycol or propylene glycol are used in the water containing part of the composition. According to the invention the water based hard core part contains the skin moisturizing agent in an amount of 0.1 to 40 percent by weight, preferably 10 to 30 percent by weight, most preferred 15 to 25 percent by weight based on the total weight of the water based hard core part.

The emollients which are formulated in the water based hard core part according to the invention are preferably ester components. Emollients are used in the cosmetic composition to provide superior lubricating properties and facilitates the deposition of colour additives. Further, the light skin feelings are impaired by increasing the slip of the composition and exhibiting a uniform spreading.

Suitable esters are isopropyl isostearate, lauryl lactate, octyl pelargonate, octyl isononanoate, diisopropyl sebacate, neopentyl glycol dioctanoate, cetyl ethylhexanoate, ethylhexyl isononanoate, neopentyl glycol diethyl hexanoate neopentyl glycol dicaprate, isodecyl oleate, isostearyl neopentanoate, cetearyl octanoate, glyceryl trioctanoate, isocetyl stearate, isostearyl isostearate, diasoproyl dimer dilineoleate, PPG-3 benzyl ether myristate, isopropyl palmitate, isopropyl miristate or octyl palmitate, most preferred isopropyl isostearate.

According to the invention the water based hard core part contains emollients in an amount of 0.1 to 40 percent by weight, preferably 5 to 25 percent by weight, most preferred 10 to 20 percent by weight based on the total weight of the water based hard core part.

Further a W/O or W/Si emulsifier is used to stabilize the water based hard core part by preventing water from escaping from the polyethylene matrix. Suitable W/O emulsifiers are polyglycerol-2 triisostearate, PEG-30 dipolyhydroxystearate, decaglyceryl heptaoleate, polyglyceryl-3 diisostearate, PEG-8 distearate, diglycerol dipolyhydroxystearate, glycerol isostearate, sorbitan isostearate, polyglyceryl-3 methylglucose distearate or steareth-2. As W/Si emulsifiers preferably cetyl PEG/PEG-10/1 dimethicone, bis-PEG/PEG-14/14 dimethicone & cyclopentasiloxane or cyclopentasiloxane & PEG-19/19 dimethicone are used according to the invention.

According to the invention only low amounts, preferably 0.1 to 10 weight %, more preferred 0.1 to 5 weight %, most preferred 0.1 to 2.5 weight % of emulsifier are sufficient to stabilize the inner core part.

The cosmetic core stick according to the invention comprises at least one water or oil soluble active ingredient in the water based hard core part. An active ingredient according to the invention is any cosmetical and/or pharmaceutical component which change the properties of the skin, in particular which change the properties of the skin positively. Suitable active ingredients are anti-aging ingredients, such as anti-wrinkles ingredients, antioxidants or cell regeneration impairing ingredients, wound-healing actives, such as anti-inflamatory ingredients or anti-acne ingredients, antimicrobially, proteolytically or keratolytically effective substances, skin lightening actives and insect repellents.

For example salicylic acid, alpha-hydroxy acids, vitamins, preferably vitamin C, vitamin H, vitamin B, vitamin E, vitamin A and derivatives thereof, such as vitamin acetate, vitamin phosphate or vitamin palmitat, tocopheryl acetate, folic acid, niacin, provitamins, and derivatives thereof, flavones or flavonoids, omega-3 fatty acid esters, algae, cucumber, aloe Vera, fruit-, root-, flower-, seed-, herbal- and maidenhair tree extracts, white lily, black-, green-, white tea, pro-glutamic acid, 2-hydroxyoctanoic acid, honey, propolis, proteins, peptides, carnithine, sulphur, coenzyme Q-10, phenolic antioxidants, such as butylated hydroxyanisol, butylated hydroxytoluene, propyl gallate or mono-tertiary-butylhydroquinone, lactic acid, glycerol ethers, such as octoxyglycerol, aluminium salts, zinc compounds, or wound healing compounds, such as dexpanthenol, zinkoxid, plant extracts, such as chamomille extracts, tea tree extracts or witch hazel extract are used according to the invention.

The release of the water or oil soluble active ingredients from the water based hard core of the invention is significantly increased compared with conventional preparations. This is highly advantageous because the active ingredients, for instance anti-acne actives or wound healing compounds incorporated into the formulation according to the invention are more effective in a lower concentration.

The water or oil soluble active is contained in the water based hard core part in an amount of 0.01 to 20 percent by weight, preferably 0.1 to 15 percent by weight, most preferred 1 to 10 percent by weight based on total weight of the water based hard core part. If pharmaceutically active ingredients are used in the cosmetic core stick the amount of the active is preferably reduced until the OTC (over-the-counter) level so that the cosmetic core stick remains available for cosmetic use.

According to the invention both parts of the cosmetic core stick contain colour additives. As a colour additive any cosmetically acceptable pigment, natural and synthetic, organic and inorganic, dyes and mixtures thereof are preferred. According to the invention pigments are white or coloured, mineral or organic particles which are intended to colour and/or opacify the composition. Examples for mineral pigments which can be used according to the invention are made of titanium dioxide, zirconium oxide or cerium oxide, zinc oxide, iron (II; III) oxide, chromium oxide or bismuth oxychloride. Further any pearlescent pigments can be used, as ferric blue, manganese violet, copper powder, bronze powder. As organic pigments the use barium, strontium, calcium and aluminium lakes is preferred. Dyes may be soluble in water or organic solvents. Preferred dyes are for example FD&C Blue #1, FD&C Yellow #5, FD&C Red 40, DC violet #2.

In another preferred embodiment nacres or goniochromatic pigments, for example multilayer interference pigments, and/or reflective pigments may be included into the cosmetic core stick. As nacres coated mica can be used according to the invention. The mica can be coated with titanium oxide, iron oxide, natural pigments or bismuth oxychloride.

The colour additive is present in an amount sufficient to impart a colour to the human skin on which it is applied. According to the invention at about 0.1 to about 30 weight % colour additive, preferably at about 0.5 to about 25 weight %, more preferred at about 1 to about 20 weight % and most preferred at about 5 to about 20 weight % are used in the anhydrous shell part and the water based hard core part. All weight % are based on the total weight of the colour containing part of the cosmetic core stick.

In another preferred embodiment the cosmetic core composition further comprises powders and/or mineral or synthetic particles of any form. Powders are used to absorb an excess of natural skin oils, to flatten small wrinkles and help to make a shine-free even look after application. In particular spherical powders are used in the anhydrous shell part. As mineral particles mica, talc, such as microtalc, muscovite, sericite, zeolite, nature cotton powder, cellulose powders, starch, such as corn starch or rice starch and derivatives thereof, carbonates, such as magnesium carbonate, calcium carbonate, silicates, such as aluminum silicate, barium silicate, calcium silicate, magnesium silicate, sulfates such as barium sulfate and magnesium sulphate, phosphates, such as calcium phosphate, metal dioxides, aluminia and mixtures thereof. Magnesium sulphate and kaolin are preferred. Synthetic particles can be made of acrylic acid polymers, such as polyacrylates, in particular polymethylmetacrylat (PMMA), methyl metacrylate crosspolymer, acrylic acid ethers, silica, polyamides, polyethylene, polyurethane, nylon-12, polystyrenes, styrene/DVB copolymer, silicone resin powder, polyalkylenes, vinyl acetates, polyvinyl pyrrolidones, silicones and mixtures thereof. In particular mixtures, such as Mica (and) Acrylates Copolymer (and) Sodium dehydroacetate (Sericite GMS-4C) are used according to the invention. Preferably nature cotton, corn starch, micro talc, methyl metacrylate crosspolymers, such as Ganzpearl GMP-0820 or Sericite GMS-4C are used as powders in the cosmetic core stick composition.

Powders are contained at about 0.1 to about 40 weight %, preferably at about 10 to about 30 weight %, most preferred at about 15 to about 20 weight %. All weight % are based on the total weight of the powder containing part of the core stick.

According to the invention any cosmetically acceptable hydrocarbon or silicone can be used as a solvent in the formulation of the anhydrous shell part of the cosmetic core stick. As hydrocarbons alkanes or isoalkanes are preferred, in particular isoparaffins, for instance isododecane, isodecane and isohexadecane or isohexyl neopentanoate and mixtures thereof. As silicone cyclopentasiloxane or polydimethylsiloxanes, such as dimethicone and derivatives thereof, for instance dimethiconol, amodimethicone, cyclomethicones, phenyl trimethicone, dimethicone copolyol, stearyl dimethicone, cetyl dimethicone and mixtures thereof are preferred. According to the invention it is especially preferred to use silicone of Dow Corning, for instance the Dow Corning 200 Fluids (silicones 200/2, silicones 200/5, silicones 200/20, silicones 200/50, silicones 200/350) or Dow Corning 245 Fluid. But also other cosmetically acceptable solvents like esters or vegetable oils may be applied.

Several physical properties of the anhydrous shell part such as texture, viscosity or melting temperature can be adjusted by choosing the appropriate solvent. For instance the viscosity of the composition is adjusted by adding different amounts of polydimethylsiloxanes. The amount of the solvent is calculated separately in order to reach 100 weight %.

Human skin is sensitive to UV-radiation induced damages. Highly sensitive areas, such as lips which do not contain any melanin pigments or regenerating and healing skin have to be well protected. Thus, the anhydrous shell part contains at least one UV-A and one UV-B filter substance. Suitable UV-A- and UV-B filters, such as organic or inorganic UV-reflectors or UV-absorbers are known in the state of the art.

Benzotriazoles, triazines, bezophenones, such as benzophenone-2, benzophenone-3 or benzophenone-4, hydroxybenzophenone derivatives, such as 2-hydroxy-4-methoxybenzophenone, 4-aminobenzoic acid, 3-(4'-trimethylammonium)benzylidene-bornane-2 -on-methylsulphate, 3,3,5-trimethylcyclohexyl-salicylate, 2-phenylbenzimidazole-5-sulfonic acid and salts thereof, 3,3'-(1,4-phenylendimethin)-bis(7,7-dimethyl-2-oxobicyclo-(2,2,1)heptane-1-methanesulfonic acid) and salts thereof, 1-(4-tert-butylphenyl)-3-(4-methoxyphenyl)propane-1,3-dione, 3-(4'-sulfo)-benzylidene-bornane-2-one and salts thereof, 2-cyan-3,3-diphenylacrylic acid-(2-ethylhexylester), N-(2(and 4)-(2-oxoborne-3-ylidenemethyl) benzyl)acrylamide polymer, 4-methoxy-cinnamic acid-2-ethyl-hexylester, PEG-25 PABA, Isoamyl p-methoxy-cinnamate, ethylhexyl methoxycinnamate, octyl-triazone, drometrizole trisiloxane, dioctyl butamido triazone, 4-methylbenzyleidene camphor, 3-benzylidene camphor, octyl salicylate, octyl dimethyl PABA, 1,1,3,3-tetramethylbutyl)phenole; 2,2'-(1,4-Phenylene) bis(1H-benzimidazol-4,6-disulfonic acid, 2,4-bis(4-(2-ethylhexyloxy)-2-hydroxyphenyl)-6-(4-methoxyphenyl)-1,3,5-triazine, dimethicodiethylbenzal-malonate, diethylamino hydroxybenzoyl hexyl benzoate; water-soluble and sulphonated organic and/or inorganic pigments, for instance inorganic oxides of zinc, titanium, iron, manganese, aluminium or cerium are used according to the invention.

The anhydrous shell part further comprises at least one organic filler. As used herein, the term organic fillers means fillers containing a single organic chemical compound or fillers containing an organic substrate coated with at least one other organic or mineral compound. Suitable fillers are for example zinc stearate, boron nitride, starch powders, modified starch powders, poly-beta-alanine powders, lauroyllysine powders, nylon powders such as Organosol™ or Nylon R™, polyethylene powders, polytetrafluoroethylene powders, such as Teflon™, copolymer microspheres such as those of polyvinylidene chloride/acrylonitrile, e.g. Expanecel™ (Nobel Industries), acrylic acid copolymers, e.g. Polytrap™ (Dow Corning), silicon resin microbeads such as Tospear™ (Toshiba) and the like.

Suitable preservatives to ensure stability over time of the core sticks according to the invention are for instance parabens, phenoxyethanol, caprylyl glycol, such as Lexgard O (Caprylyl Glycol) , Lexgard GMCY (Glyceryl Caprylate) or any typical preservative used in the personal care/cosmetic industry. For example esters are also known for their antiseptic properties and they are conventionally used in this respect in compositions intended to be administered to man or animals as pharmaceuticals, nutritionals and cosmetic compositions.

The anhydrous shell part further comprises a cosmetically acceptable surfactant. Suitable surfactants are widely known to a person skilled in the art. Preferred surfactants are Sorbitan Sesquioleate , Lecithin , Laureth-7, Lauryl Phosphate, Oleth-10, Polysorbate 85, Cocamidopropyl Betaine.

The dimensions of the whole core stick according to the invention can be varied from 20 to 30 mm along the length axis. The length depends on the packaging and also on the specific mold adapted to the pack. The diameter of the core stick is in the range of 10 to 30 mm. Preferably the length of the core stick is bigger than the diameter. The relation of the diameter of the water based hard core part to the anhydrous shell part depends on the amounts of both parts based on the whole core stick.

In another embodiment of the invention the anhydrous shell part is present in a range from 15 to 85 percent by weight and the water based hard core part is present in a range from 85 to 15 percent by weight based on total weight of cosmetic core stick. In a preferred embodiment of the invention the amount of the water based hard core part is more than 50 weight %, more preferred more than 60 weight %, most preferred more than 70 weight % based on the total amount of the cosmetic core stick according to the invention.

The preferred cosmetic core stick of the invention is a solid concealer stick being a colour foundation with superior application and providing superior care benefits due to the incorporation of both oil soluble and water soluble active ingredients in the water based hard core part. The product provides excellent comfort, soft focus effect and oil absorption throughout wear all day long combined with practical and comfort provided skin. The main role of the shell part is to provide colour to the skin and hide imperfections. The main role of the core part is to prevent transepidermal water loss and make skin stays softer and smoother, more hydrated and more elastic.

The anhydrous shell part is a cream to powder formula containing waxes, polyethylene, skin conditioning agents, spherical powder, esters, surfactant-emulsifying agents, alcohols, colour additives, carboxylic acids, carbohydrates, inorganic compounds, siloxanes and silanes. Preferably at least one sunscreen agent is included to shield the skin against UV damage.

The water based hard core part is a unique solid emulsion which allows the incorporation of water soluble actives. The internal part is composed of water, polyethylene, esters, alcoxylated carboxylic acids, glyceryl esters and derivatives, alcohols, biological polymers and derivatives, carboxylic acids, inorganic salts, siloxanes and silanes.

It is another object of the present invention to provide a method for producing the cosmetic core stick of the invention.

The anhydrous shell part is prepared in a per se known manner. In a preferred embodiment the at least one wax component, the polyethylene, the at least one skin conditioning agent and the at least one surfactant are added into an appropriate flask. The ingredients are heated to 80 to 85 °C under continuous stirring until the components are completely molten. After a homogeneous mixture is achieved the temperature can be reduced to 75 °C, but the mixture does not start to solidify again. The initial over-heating is needed to ensure that all components are completely molten and can be distributed equally. The at least one spherical powder, the at least one colour additive, the at least one UV-filter substance and the at least one organic filler are added stepwise to the molten mass. Thereby the mixture is stirred or mixed continuously by 75 °C. Optionally the ingredients can be premixed before adding. If cosmetically and/or pharmaceutically auxiliary substances should be included into the mixture they are added last. The mixture is mixed until homogeneity, but at least for 15 minutes.

The water based hard core part is prepared stepwise. The oily and the aqueous phase are prepared separately before mixing. For the oily phase of the water based hard core part the at least one emollient, the polyethylene and the at least one W/O and/or W/Si emulsifier are mixed and heated to 80 to 83 °C initially. After a homogeneous mixture is achieved the mixture is cooled down to 55 to 60 °C. The at least one colour additive and the at least one preservative are added under continuous stirring. If oil soluble cosmetically or pharmaceutically active ingredients are comprised in the water based hard core part, they are added last to minimize shearing and heating of the active ingredients. The oily phase is finally mixed for at least 20 to 25 minutes. If the dispersion does not look homogeneously it is mixed for another 5 minutes. The oily phase is stored until use at 55 to 60 °C and stirred occasionally.

For the aqueous phase of the water based hard core part the at least one skin moisturizing agent and the at least one cosmetically or pharmaceutically water soluble active are added to the water and mixed until homogeneity. Optionally cosmetically or pharmaceutically auxiliary substances can be added. The resulting aqueous mixture is heated to the temperature of the oily phase, preferably to 55 to 60 °C.

If both phases are tempered and homogeneously mixed the aqueous phase is slowly added into the oily phase under continuous stirring. The resulting emulsion represents the water based hard core part according to the invention. The emulsion is very stable and does not separate into the two phases even if the temperature is reduced. All added solid substance are distributed homogeneously. Optionally further colour additives, plant extracts or cosmetically and/or pharmaceutically auxiliary substances can be added.

The stick according to the invention is prepared by pouring the anhydrous shell part and the water based hard core part into an appropriate mold. First the molten mass of the shell part is poured into the cavity of the mold. A removable rod is placed into the middle of the mass to achieve a second cavity for the water based core part. After cooling the mass down until solidification, the removable rod is taken out so that a hollow appears in the middle of the stick. The molten water based hard core part is poured into the hollow and after removing the excess of the mass from the top of the mold the mass of the water based hard core part is cooled down until solidification. Due to the higher melting point the anhydrous shell part remains solid during the pouring of the water based hard core part. According to the invention the solid stick can be removed from the mold and used immediately.

It is another object of the present invention to provide a method for healing and caring for biological surfaces, in particular human skin, by applying the cosmetic core stick composition of the invention.

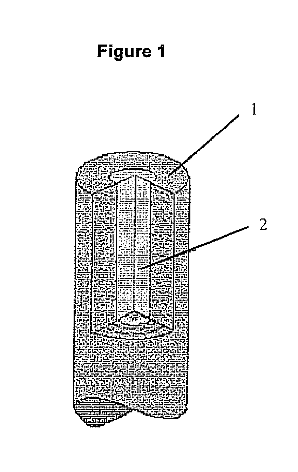

Figure 1 shows a section view of a cosmetic core stick according to the invention. The anhydrous shell part 1 covers the water based hard core part 2.

The following examples are offered to illustrate the cosmetic core stick of the present invention. They are not intended to be limiting in any respect.

### Example 1

A cosmetic core stick according to the invention is prepared wherein the shell part of the composition is prepared by mixing the phases A to D which comprise:

| Phase A: | | |
|---|---|---|
| 0.1 - 8.9 | weight % | Waxe(s) |
| 0.1 - 12.0 | weight % | Polyethylene |
| 0.1 - 35.0 | weight % | Emollient(s) |
| 0.1 - 8.9 | weight % | Silicone(s) |
| 0.2 - 2.0 | weight % | Emulsifier(s) |
| 0.1 - 10.0 | weight % | UV-filter(s) |
| 0.1 - 3.0 | weight % | Active ingredient(s) |
| 0.1 - 2.0 | weight % | Preservative(s) |

| Phase B: | | |
|---|---|---|
| 0.1 - 20.0 | weight % | Powder(s) |

| Phase C: | | |
|---|---|---|
| 0.1 - 17.5 | weight % | Colour additive(s) |
| 0.1 - 20.0 | weight % | Powder(s) |

| Phase D: | | |
|---|---|---|
| 0.1 - 25.0 | weight % | Siloxane(s) |
| 0 - 0.5 | weight % | Fragrance(s) |

The external shell part is prepared by adding the ingredients of Phase A into a sealed and explosion proofed kettle equipped with a lightning or similar typed mixer. The mixture is heated to 80 to 85 °C and mixed mechanically until it is completely molten. Then the temperature is decreased to 75 °C and Phase B is added gradually under continuous mixing. After Phase B is added completely the mixture is mixed for at least 15 minutes or until uniform. Next the ingredients of Phase C are preblended and added to the kettle. The mixture is further mixed/homomixed for at least 30 minutes or until the pigments are completely wetted out. If the dispersion does not look good, it has to be mixed for another 10 minutes. Finally the Phase D ingredients are added and the mixture is dropped to 75 - 78 °C under continuous mixing. The resulting composition of the shell part is poured into the mold with removable rod is taken into the middle of the mold and the mass is cooled down until solidification.

**The water based hard core part of the composition is comprise:**

| Phase A: | | |
|---|---|---|
| 0.1 - 3.0 | weight % | Powder(s) |
| 5.0 - 25.0 | weight % | Emollient(s) |
| 5.0 - 20.0 | weight % | Polyethylene |
| 0.1 - 4.5 | weight % | Emulsifier(s) |
| 0.1 - 3.0 | weight % | Active ingredient(s) |

| Phase B: | | |
|---|---|---|
| 0.1 - 1.5 | weight % | Colour Additive(s) |
| 0.1 - 2.0 | weight % | Powder(s) |

| Phase C: | | |
|---|---|---|
| 0.1 - 15.0 | weight % | Skin moisturing agent(s) |
| 0.1 - 85.0 | weight % | Water |
| 0.1 - 2.0 | weight % | auxiliary agent(s) |
| 0.1 - 2.0 | weight % | Active agent(s) |
| 0.1 - 1.5 | weight % | Preservative(s) |

| Phase D: | | |
|---|---|---|
| 0.1 - 0.5 | weight % | Colour additive(s) |
| 0.1 - 1.0 | weight % | Active agent(s) |

The water based hard core part is prepared by mixing all the ingredients of Phase A in the main cattle and the mixture is heated to 80 to 85 °C. After initial heating the batch is cooled to 65 to 68 °C. Phase B ingredients are added under continuous mixing/homomixing for at least 20 to 25 minutes or until uniform. If the dispersion does not look homomixed, it has to be mixed for another 5 minutes.

The skin moisturing agents of Phase C are premixed until uniform. When uniform the remaining Phase C ingredients are added. Then the water Phase C is heated to 68 °C and slowly given to the oil phase (A-B) under continuous mixing with a constant mixing rate. The homogeneous emulsion is mixed for another 5 minutes. Ingredients of Phase E are added until gently mixing. Finally the mixture is stirred for further 5 minutes. The resulting composition of the core stick is poured into the hollow appeared in the middle of the shell stick after taking out the removable rod. The excess of the mass is removed from the top of the mold and the remaining mass is cooled down until solidification.

### Example 2

A cosmetic core stick according to the invention is prepared as shown in example 1. Phases A to D of the shell part of the composition comprise:

| Phase A: | | |
|---|---|---|
| **Amount [weight %]** | **Trade names** | **INCI names** |
| 0.1 - 8.9 | Ozokerite wax 7726 | Ozokerite |
| 0.1 - 12.0 | Jeenate 3H | Polyethylene |
| 0.1 - 8.9 | Silicone Fluid 200/350 | Dimethicone |
| 0.1 - 5.0 | Parsol MCX | Ethylhexyl Methoxycinnamate |
| 0.2 - 2.0 | Arlacel 83V | Sorbitan Sesquiolate |
| 0.1 - 10.0 | Jeechem CEO | Cetearyl Octanoate |
| 0.1 - 25.0 | Schercemol NGDO | Neopentyl Glycol |
| | | Diethylhexanoate |
| 0.1 - 2.0 | Lexgrad O | Caprylyl Glycol |
| 0.1 - 1.0 | Vitamin E acetate | Vitamin E Acetate |
| 0.1 - 5.0 | Z-cote | Zinc Oxide |
| 0.1 - 2.0 | Salicylic acid extra pure | Salicylic Acid |

| Phase B: | | |
|---|---|---|
| **Amount [weight %]** | **Trade names** | **INCI names** |
| 0.1 - 10.0 | Ganzpearl GMP-0820 | Methyl Metacrylate Crosspolymer |
| 0.1 - 10.0 | Globe Mz Star 03401 | Corn Starch |

| Phase C: | | |
|---|---|---|
| **Amount [weight %]** | **Trade names** | **INCI names** |
| 0.1 - 10.0 | C47-056 Cos white | Titanium Dioxide |
| 0.1 - 2.5 | Red iron oxide C332199 | Iron Oxide |
| 0.1 - 2.5 | Blk iron oxide C335000 | Iron Oxide |
| 0.1 - 2.5 | Yel iron oxide C331700 | Iron Oxide |
| 0.1 - 20.0 | Microtalc It Extra | Talc |

| Phase D: | | |
|---|---|---|
| **Amount [weight %]** | **Trade names** | **INCI names** |
| 0.1 - 25.0 | Dowcorning 245 fluid | Cyclopentasiloxane |
| 0 - 0.5 | Fragrance(s) | Fragrance(s) |

Phases A to D of the water based hard core part of the composition comprise:

| Phase A: | | |
|---|---|---|
| **Amount [weight %]** | **Trade names** | **INCI names** |
| 0.1 - 3.0 | Aerosil R972 | Silica Dimethyl Silylate |
| 5.0 - 25.0 | Wickenol 131 VEG | Isopropyl lsostearate |
| 5.0 - 20.0 | Jeenate 3H | Polyethylene |
| 0.1 - 2.0 | Arlacel P135 | PEG-30 Dipolyhydroxystearate |
| 0.1 - 2.5 | Prisorine 3793 | Polyglyceryl-2 Triisostearate |
| 0.1 - 1.0 | Vitamin E acetate | Tocopheryl Acetate |
| 0.1 - 2.0 | Salicylic acid extra pure | Salicylic Acid |

| Phase B: | | |
|---|---|---|
| **Amount [weight %]** | **Trade names** | **INCI names** |
| 0.1 - 1.0 | Titanium dioxide | Titanium Dioxide |
| 0.1 - 0.5 | DC Violet #2 K7014 | Violet 2 |
| 0.1-0.5 | Nature cotton | Gossypium Herbaceum |
| | | (Cotton) |
| | | Mica (and) Acrylates |
| 0.1 - 1.5 | Sericite GMS-4C | Copolymer (and) Sodium |
| | | Dehydroacetate |

| Phase C: | | |
|---|---|---|
| **Amount [weight %]** | **Trade names** | **INCI names** |
| 0.1 - 15.0 | Propylene glycol | Propylene Glycol |
| 0.1 - 1.0 | Hyacare | Sodium Hyaluronate |
| 0.1 - 85.0 | Deionised water | Deionised Water |
| 0.1 - 1.0 | Mag sulfate hepta | Magnesium Sulfate |
| 0.1 - 1.0 | AA-2G | Ascorbyl Glucoside |
| 0.1 - 1.0 | Trilon BD | Disodium EDTA |
| 0.1 - 1.0 | Unitamuron H-22 | Pentylen Glycol (and) Tamarindus Indica Extract |
| 0.1 - 1.5 | Lexgard O | Caprylyl Glycol |

| Phase D: | | |
|---|---|---|
| **Amount [weight %]** | **Trade names** | **INCI names** |
| 0.1 - 0.5 | Colorona dark blue | Mica & Titanium Dioxide & |
| | | Ferric Ferrocyanide |
| 0.1- 1.0 | ABS Chamomille & | Chamomille & Cucumber |
| | Cucumber Extract PF | Extract |

### Example 3

Storage stability of cosmetic compositions is very important. To determine the storage stability of the cosmetic core stick according to the invention a stress test was performed. The cosmetic core stick was stored at room temperature, at 5° C, at 37° C, at 45° C and at 45° C with 50% of relative humidity, under daylight and UV radiation. After 8 weeks of standard for cosmetic industry stability tests both parts of the core stick of the invention do not show any changes in texture, colour, odour or viscosity. Water or oil separation was not observed. The skin feeling remains smooth and soft.

## Claims

1. Cosmetic core stick consisting of
a) an anhydrous shell part comprising at least one wax component, polyethylene, at least one skin conditioning agent, at least one colour additive, at least one spherical powder, at least one UV filter substance, at least one organic filler and at least one surfactant
and
b) a water based hard core part which is a solid W/O or W/Si emulsion comprising water, polyethylene, at least one emollient, at least one cosmetically or pharmaceutically water and/or oil soluble active, at least one W/O or W/Si emulsifier, at least one skin moisturizing agent, at least one colour additive and at least one preservative.

2. Cosmetic core stick according to claim 1, wherein the anhydrous shell part and/or the water based hard core part comprise cosmetically acceptable auxiliary substances.

3. Cosmetic core stick according to claims 1 or 2 , wherein the cosmetically acceptable auxiliary substances comprise powders, such as absorbers or thickening agents, consistency-imparting agents, such as viscosity control agents, fillers, film forming agents, solvents, preservatives, fragrances, such as perfume oils, sun filters, photoreactive agents, stabilizers, repellents, wound healing substances and mixtures thereof.

4. Cosmetic core stick according any one of claims 1 to 3, wherein the water based hard core part has a melting point from 45 °C to 65 °C, preferably from 50 °C to 60 °C and most preferred from 55 °C to 58 °C.

5. Cosmetic core stick according any one of claims 1 to 4 wherein the skin conditioning agent is selected from the group of vitamins, mineral salts, trace elements, plant extracts, animal extracts, proteins, enzymes and mixtures thereof.

6. Cosmetic core stick according to any one of claims 1 to 5, wherein the skin moisturizing agent is an occlusive moisturizer, preferably a silicone, such as dimethicone or cyclomethicone and/or a humectant agent, preferably glycerol, sorbitol, mannitol, polypropylene glycol, dipropylene glycol, polyethylene glycol, butylene glycol, chitosan, fucogel, glycin, alpha-hydroxy acids, such as lactic acid, glycolic acid, glutamic acid, glucoronic acid, sodium pyrolidon carboxylic acid, hyaluronic acid and salts thereof, ascorbyl glucoside, pentylene glycole (and) Tamarindus Indica Extract, urea and/or salts of the alkaline metals or the alkaline earth metals and mixtures thereof.

7. Cosmetic core stick according to any one of claims 1 to 6, wherein the emollient is an ester component, preferably isopropyl isostearate, lauryl lactate, octyl pelargonate, octyl isononanoate, diisopropyl sebacate, neopentyl glycol dioctanoate, cetyl ethylhexanoate, neopentyl glycol dicaprate, isodecyl oleate, isostearyl neopentanoate, cetearyl octanoate, glyceryl trioctanoate, isocetyl stearate, isostearyl isostearate, diisopropyl dimer dilineoleate, PPG-3 benzyl ether myristate, isopropyl palmitate, isopropyl miristate or octyl palmitate, most preferred isopropyl isostearate.

8. Cosmetic core stick according to any one of claims 1 to 7, wherein the W/O emulsifier is polyglycerol-2 triisostearate, PEG-30 dipolyhydroxystearate decaglyceryl heptaoleate, polyglyceryl-3 diisostearate, PEG-8 distearate, diglycerol dipolyhydroxystearate, glycerol isostearate, sorbitan isostearate, polyglyceryl-3 methylglucose distearate or steareth-2 and the W/Si emulsifier is cetyl PEG/PEG-10/1 dimethicone, bis-PEG/PEG-14/14 dimethicone & cyclopentasiloxane, cyclopentasiloxane & PEG-19/19 dimethicone.

9. Cosmetic core stick according to any one of claims 1 to 8, wherein the water and/or oil soluble active is selected from the group consisting of salicylic acid, vitamins, preferably vitamin C, vitamin H, vitamin B, vitamin E, vitamin A, and derivatives thereof, omega-3 fatty acid esters, folic acid, niacin, provitamins and derivatives thereof, algae, chamomile, cucumber, aloe Vera, fruit-, root-, flower-, seed-, herbal-, witch hazel and maidenhair tree extracts, white lily, black-, green-, white tea, pro-glutamic acid, 2-hydroxyoctanoic acid, honey, proteins, peptides, sulphur, carnithine, coenzyme Q-10, phenolic antioxidants, such as butylated hydroxyanisol, butylated hydroxytoluene, propyl gallate or mono-tertiary-butylhydroquinone, lactic acid, glycerol ethers, such as octoxyglycerol, aluminium salts, zinc compounds, dexpanthenol and mixtures thereof.

10. Cosmetic core stick according to any one of claims 1 to 9, wherein the anhydrous shell part contains the polyethylene in an amount of 0.1 to 35 percent by weight, preferably 8 to 15 percent by weight based on total weight of the anhydrous shell part.

11. Cosmetic core stick according to any one of claims 1 to 10, wherein the water based hard core part contains more than 50 percent by weight, preferably more than 60 percent by weight, more preferred more than 70 percent by weight and most preferred more than 80 percent by weight of water based on total weight of the water based hard core part.

12. Cosmetic core stick according to any one of claims 1 to 11, wherein the water based hard core part contains the polyethylene in an amount of 0.1 to 30 percent by weight, preferably 1 to 20 percent by weight, most preferred 5 to 15 percent by weight based on total weight of the water based hard core part.

13. Cosmetic core stick according to any one of claims 1 to 12, wherein the water based hard core part contains the skin moisturizing agent in an amount of 0.1 to 40 percent by weight, preferably 10 to 30 percent by weight, most preferred 15 to 25 percent by weight based on total weight of the water based hard core part.

14. Cosmetic core stick according to any one of claims 1 to 13, wherein the water based hard core part contains the water or oil soluble active in an amount of 0.01 to 20 percent by weight, preferably 0.1 to 15 percent by weight, most preferred 1 to 10 percent by weight based on total weight of the water based hard core part.

15. A method for producing the cosmetic core stick of claims 1 to 14, wherein
a) the anhydrous shell part is prepared in a known manner;
b) the oily phase of the water based hard core part is prepared by mixing the at least one emollient with the polyethylene and the at least one W/O and/or W/Si emulsifier and heating the mixture to 80 to 83 °C, after cooling to 55 to 60 °C the at least one colour additive-and the at least one preservative and the at least one cosmetically and/or pharmaceutical oil soluble active is added;
c) the aqueous phase of the water based hard core part is prepared by mixing the at least one skin moisturizing agent, the at least one cosmetically or pharmaceutically water soluble active and optionally cosmetically or pharmaceutically auxiliary substances into the water and heating the mixture to 55 ° to 60 °C;
d) the water based hard core part is prepared by adding the aqueous phase prepared in step c) slowly into the oily phase prepared in step b) under continuous mixing, optionally further colour additives, plant extracts and/or cosmetically and/or pharmaceutically auxiliary substances can be added; and finally
e) the core stick is prepared by pouring the molten mass of step a) into the cavity of a mold, taking a removable rod into the middle of the mold and cooling the mass down until solidification, after cooling the removable rod is taken out so that a hollow appears in the middle of the stick, then the molten mass of step d) is poured into the hollow, the excess of the mass is removed from the top of the mold, and the remaining mass is cooled down until solidification.
